# EUROPÄISCHE PATENTSCHRIFT

(11) **EP 2 364 665 B1**
(45) Veröffentlichungstag und Bekanntmachung des Hinweises auf die Patenterteilung: **08.02.2012**
(21) Anmeldenummer: 10155736.1
(22) Anmeldetag: 08.03.2010
(51) Int. Cl.: A61B 19/00, A61C 19/00

(54) **Reinigungs- oder Pflegegerät für medizinische Instrumente**
Cleaning or maintenance device for medical instruments
Dispositif de nettoyage et d'entretien pour instruments médicaux

(43) Veröffentlichungstag der Anmeldung: 14.09.2011
(73) Patentinhaber: W & H Dentalwerk Bürmoos GmbH, 5111 Bürmoos (AT)
(72) Erfinder: Pfaffinger, Nikolaus, 5120, St. Pantaleon (AT); Spieler, Christian, 5152, Michaelbeuern (AT)
(74) Vertreter: Benda, Ralf

(56) Entgegenhaltungen:
- EP-A1- 1 683 499
- WO-A1-2010/016066
- US-A1- 2006 196 728
- US-A1- 2007 031 778

## Beschreibung

Die vorliegende Erfindung betrifft ein Reinigungs- oder Pflegegerät zur Reinigung oder Pflege medizinischer, insbesondere dentaler, Instrumente und ein entsprechendes Reinigungs- oder Pflegeverfahren.

Ein derartiges Reinigungs- oder Pflegegerät ist zum Beispiel aus der Patentanmeldung US 2007/0031778 A1 bekannt. Es umfasst eine Reinigungskammer, in der zwei oder mehr Anschlüsse für die zu reinigenden Instrumente vorgesehen sind. Durch das Vorsehen von zwei oder mehr Anschlüssen können in vorteilhafter Weise während eines Reinigungs- oder Pflegezykluses mehrere Instrumente gereinigt oder gepflegt werden.

Der Nachteil dieses Reinigungs- oder Pflegegeräts liegt darin, dass mit jedem zusätzlichen Anschluss der Aufbau des Reinigungs- oder Pflegegeräts und die Steuerung der Reinigungs- oder Pflegeverfahren komplizierter werden, da jeder Anschluss zum Beispiel über eigene Versorgungsvorrichtungen, insbesondere über eigene Leitungen oder Leitungsabschnitte, mit den Quellen der Reinigungs-, Pflege- und / oder Spülmittel verbunden werden muss oder da für jeden Anschluss eigene Stellmittel, Steuermittel oder Sensoren benötigt werden. Damit nimmt in nachteiliger Weise auch das Volumen der Reinigungs- oder Pflegegeräte zu, so dass Reinigungs- oder Pflegegeräte mit mehreren Anschlüssen für die zu reinigenden Instrumente oftmals sehr voluminös und sperrig sind.

US 2006/0196728 offenbart ein Reinigungsgerät für dentale Instrumente mit den Merkmalen des Oberbegriffs von Anspruch 1.

Die Patentanmeldung WO 2010/016066 A1 offenbart ein Gerät zur Oberflächenbeschichtung dentaler Restorationskörper, insbesondere zum Beschichten von Inlays, Kronen, Brücken, Verblendungen etc. mit Keramik-, Metall- oder Compositbeschichtungen. Das Gerät weist Halterungen zum Befestigen der Restorationskörper sowie mehrere Düsen zur Abgabe von Medien auf die Oberfläche der Restorationskörper auf. Die Düsen und Halterungen sind relativ zueinander beweglich.

Der vorliegenden Erfindung liegt daher die Aufgabe zugrunde, ein Reinigungs- oder Pflegegerät zur Reinigung oder Pflege medizinischer, insbesondere dentaler, Instrumente zu schaffen, das unter Beibehaltung des Vorteils, dass während eines Reinigungs- oder Pflegezykluses mehrere Instrumente gereinigt oder gepflegt werden können, einen einfacheren Aufbau der Versorgungsvorrichtung aufweist und das einen geringeren Platzbedarf hat.

Diese Aufgabe wird gemäß einem Ausführungsbeispiel durch ein Reinigungs- oder Pflegegerät zur Reinigung oder Pflege medizinischer, insbesondere dentaler, Instrumente nach Anspruch 1 und durch ein Reinigungs- oder Pflegeverfahren nach Anspruch 13 gelöst.

Gemäß unterschiedlichen Ausführungsbeispielen sind entweder die zumindest zwei Anschlüsse relativ zur Versorgungsvorrichtung und / oder ist die Versorgungsvorrichtung relativ zu den zumindest zwei Anschlüssen bewegbar.

Aufgrund der Möglichkeit die zwei oder mehr Anschlüsse und die Versorgungsvorrichtung relativ zueinander zu bewegen und somit die Anschlüsse nacheinander mit der Versorgungsvorrichtung zu verbinden oder nacheinander mit dem oder den Reinigungs- oder Pflegemitteln zu versorgen, ist es somit nicht mehr notwendig, jeden Anschluss mit eigenen Versorgungsvorrichtungen, insbesondere eigenen Leitungen oder Leitungsabschnitten, Stellmitteln, Steuermitteln, Sensoren, etc. zu versehen. Stattdessen kann die Anzahl zumindest eines dieser Elemente geringer sein als die Anzahl der Anschlüsse. Des Weiteren umfasst das Reinigungs- oder Pflegegerät oder die Versorgungsvorrichtung eine einzige Schnittstelle oder Übergabestelle zur wahlweisen Kopplung oder Verbindung mit einem der zumindest zwei Anschlüsse, an der das oder die Reinigungs- oder Pflegemittel übergeben werden. An dieser Schnittstelle sind mehrere Leitungen für Reinigungs- oder Pflegemittel vorgesehen oder zusammengefasst Bevorzugt weist die Schnittstelle zumindest eine Öffnung für ein Reinigungsfluid und zumindest eine Öffnung für ein Schmiermittel auf.

Gemäß einem bevorzugten Ausführungsbeispiel weist das Reinigungs- oder Pflegegerät eine einzige Versorgungsvorrichtung auf, mit der jeweils ein Anschluss verbunden ist, während alle anderen Anschlüsse nicht mit der Versorgungsvorrichtung verbunden sind.

Als Reinigungs- oder Pflegegerät im Sinne des vorliegenden Schriftsatzes wird jede Vorrichtung bezeichnet, die zumindest ein Reinigungs- und/oder Pflegemittel an ein medizinisches, insbesondere dentales, Instrument abgibt, um dieses zu reinigen, zu desinfizieren, zu sterilisieren, von Verschmutzungen oder Mikroorganismen zu befreien oder zu pflegen. Als Reinigungs- oder Pflegemittel können zum Beispiel eine Flüssigkeit, insbesondere Heiß- oder Kaltwasser, Dampf, Desinfektions- oder Sterilisationsmittel, ein Gas, zum Beispiel Druckluft, oder ein Schmiermittel, zum Beispiel natürliche oder synthetische Schmieröle, verwendet werden.

Als zu reinigende medizinische, insbesondere dentale, Instrumente werden insbesondere Hand- und Winkelstücke zum Antrieb verschiedenster Werkzeuge, wie rotierender Bohrer, Zahnsteinentfernungswerkzeuge, Feilen, Sägen, Reamer usw., Handstücke zur Abgabe von medizinischen Materialien, wie zum Beispiel Füllmittel oder Anästhetika, Funktionshandstücke zur Abgabe von Licht, Wasser oder Luft und nicht angetriebene Handinstrumente, wie zum Beispiel Endoskope und dergleichen verstanden.

Gemäß einem Ausführungsbeispiel sind die zumindest zwei Anschlüsse auf einem gemeinsamen Basiselement angeordnet, das relativ zur Versorgungsvorrichtung bewegbar und derart positionierbar ist, dass wahlweise einer der zumindest zwei Anschlüsse mit der Versorgungsvorrichtung verbunden ist, während ein anderer der zumindest zwei Anschlüsse nicht mit der Versorgungsvorrichtung verbunden ist. Besonders bevorzugt ist das Basiselement als Drehelement ausgebildet, das relativ zur Versorgungsvorrichtung verdrehbar ist. Das Vorsehen eines bewegbaren oder drehbaren Basiselements ermöglicht eine besonders kompakte und Platz sparende Konstruktion des Reinigungs- oder Pflegegeräts.

Gemäß einem alternativen Ausführungsbeispiel sind die zumindest zwei Anschlüsse im Wesentlichen auf einer Linie angeordnet und die Versorgungsvorrichtung oder Teile davon, insbesondere die mit den Anschlüssen verbindbaren Kupplungsstücke, sind entlang dieser Linie bewegbar, wozu bevorzugt ein Linearmotor vorgesehen ist.

Gemäß einem Ausführungsbeispiel ist das Reinigungs- oder Pflegegerät als automatisiertes Reinigungs- oder Pflegegerät mit einem Antriebsmotor zur Relativbewegung der zumindest zwei Anschlüsse und / oder der Versorgungsvorrichtung ausgebildet. Bevorzugt ist der Antriebsmotor außerhalb der Reinigungs- oder Pflegekammer angeordnet. Er umfasst zum Beispiel einen Elektromotor, insbesondere einen Schrittmotor. Die Verwendung eines Schrittmotors ist aufgrund seiner Zuverlässigkeit und einfachen Steuerung für die vorliegende Anwendung von besonderem Vorteil. Besonders bevorzugt ist der Antriebsmotor über zumindest ein Antriebselement, zum Beispiel einen Riemenantrieb und / oder eine Welle, mit den zumindest zwei Anschlüssen und / oder der Versorgungsvorrichtung und / oder dem Basiselement verbunden.

Gemäß einem anderen Ausführungsbeispiel umfasst das Reinigungs- oder Pflegegerät ein zwischen dem bewegbaren oder drehbaren Basiselement und der Versorgungsvorrichtung vorgesehenes Verteilerelement zum Leiten eines Reinigungs- und/oder Pflegemittels von der Versorgungsvorrichtung zu den zumindest zwei Anschlüssen, wobei das Verteilerelement ein erstes, mit dem Basiselement relativ zur Versorgungsvorrichtung bewegbares Verteilerstück und ein zweites, relativ zur Versorgungsvorrichtung festes Verteilerstück aufweist. Dieser Aufbau gewährleistet eine besonders zuverlässige Übergabe der Reinigungs- und/oder Pflegemittel an das Basiselement. Das erste Verteilerstück weist bevorzugt zumindest jeweils eine eigene Leitung oder einen eigenen Leitungsabschnitt für jeden der zumindest zwei Anschlüsse auf. Das zweite Verteilerstück umfasst zumindest eine Leitung für ein Reinigungs- und/oder Pflegemittel, wobei durch das Bewegen des Basiselements und somit des ersten Verteilerstücks relativ zum zweiten Verteilerstück die Leitungen des ersten Verteilerstücks mit der zumindest einen Leitung des zweiten Verteilerstücks verbindbar sind. Über diese fluidleitende Verbindung können somit das oder die Reinigungs- oder Pflegemittel von der Versorgungsvorrichtung auf die Anschlüsse und auf die jeweils mit den Anschlüssen gekuppelten, zu reinigenden Instrumente übertragen werden.

Ist das Basiselement als Drehelement ausgebildet, dann ist es gemäß einem bevorzugten Ausführungsbeispiel von besonderem Vorteil, das erste und zweite Verteilerstück scheibenförmig zu formen, wobei jedes Verteilerstück eine Außenmantelfläche und zwei Deckflächen aufweist und wobei die beiden Verteilerstücke mit jeweils einer Deckfläche aneinander gleitend angeordnet sind. Die beiden scheibenförmigen Verteilerstücke bilden somit ein Gleitlager, an dem das Drehelement drehbar gelagert ist.

Gemäß einem weiteren Ausführungsbeispiel ist an dem Reinigungs- oder Pflegegerät eine Detektionseinheit zur Positionierung des Basiselements in einer vorbestimmten Startposition vorgesehen. Die Detektionseinheit weist bevorzugt eine Markierung an einem den Antriebsmotor mit dem Basiselement verbindenden Antriebselement und einen die Markierung erkennenden Sensor auf. Eine derartige Detektionseinheit ist insbesondere dann von Vorteil, wenn das Basiselement manuell verdrehbar ist, um zum Beispiel das Kuppeln der zu reinigenden Instrumente an die Anschlüsse zu vereinfachen. Nach dem Kuppeln der Instrumente nimmt das Basiselement eine zufällige oder undefinierte Position ein. Um im folgenden Reinigungs- oder Pflegeverfahren einen der Anschlüsse fluidleitend mit der Versorgungsvorrichtung verbinden zu können, wird das Basiselement mittels der Detektionseinheit zuerst in die vorbestimmte Startposition bewegt und von dieser Startposition ausgehend dann einer der Anschlüsse mit der Versorgungsvorrichtung gekuppelt. Somit ist eine zuverlässige Verbindung der Anschlüsse mit der Versorgungsvorrichtung gewährleistet, ohne dass dazu ein hoher regeltechnischer Aufwand notwendig wäre.

Die Detektionseinheit umfasst zum Beispiel eine Strahlungsquelle, insbesondere eine Lichtquelle, einen als Markierung dienenden Fortsatz an einer Welle des Antriebsmotors und einen optischen Sensor, der ein Abdecken der Lichtquelle durch den Fortsatz detektiert, wobei das Abdecken der Lichtquelle die Startposition markiert. Gemäß einem anderen Beispiel sind die Markierung als Magnetelement an einer Welle des Antriebsmotors und der Sensor als Magnetsensor ausgebildet.

Gemäß einem Ausführungsbeispiel umfasst das Reinigungs- oder Pflegegerät eine Detektionseinheit zur Bestimmung welcher der zumindest zwei Anschlüsse mit einem medizinischen, insbesondere dentalen, Instrument belegt ist und / oder welcher der zumindest zwei Anschlüsse nicht mit einem medizinischen, insbesondere dentalen, Instrument belegt ist und zur Abgabe entsprechender Belegungssignale für jeden der zumindest zwei Anschlüsse, wobei die Detektionseinheit mit einer Steuervorrichtung verbunden ist, die auf Basis der von der Detektionseinheit abgegebenen Belegungssignale dazu ausgebildet ist, nur jene der zumindest zwei Anschlüsse mit Reinigungs- oder Pflegemittel zu versorgen, an denen ein medizinisches, insbesondere dentales, Instrument angeschlossen ist. Die Detektionseinheit besteht zum Beispiel aus einer Strahlungsquelle, insbesondere einer Lichtquelle, die eine Strahlung in die Reinigungs- oder Pflegekammer abgibt, und einem optischen Sensor, der eine Schwächung oder Unterbrechung der in die Reinigungs- oder Pflegekammer abgegebenen Strahlung durch ein an einen Anschluss gekuppeltes Instrument erkennt. Ist ein Anschluss leer, d.h. nicht mit einem Instrument belegt, dann betreibt gemäß einem bevorzugten Ausführungsbeispiel die Steuerung den Antriebsmotor derart, dass dieser nicht stoppt, wenn der leere Anschluss mit der Versorgungsvorrichtung verbunden ist, sondern dass er weiterläuft, so dass das Basiselement weiter bewegt wird, bis der nächste mit einem Instrument belegte Anschluss mit der Versorgungsvorrichtung verbunden ist.

Gemäß einem anderen Ausführungsbeispiel sind am Reinigungs- oder Pflegegerät von den zumindest zwei Anschlüssen unabhängige oder getrennt angeordnete Düsen zur Abgabe eines Reinigungs- und/oder Pflegemittels auf die Außenseite der medizinischen, insbesondere dentalen, Instrumente vorgesehen. Bevorzugt sind die zumindest zwei Anschlüsse dazu ausgebildet, ein Reinigungs- oder Pflegemittel in das Innere der medizinischen, insbesondere dentalen, Instrumente zu leiten.

Ein Verfahren zur Reinigung oder Pflege medizinischer, insbesondere dentaler, Instrumente, mit einem in der vorliegenden Schrift beschriebenen Reinigungs- oder Pflegegerät ist dadurch definiert, dass einer der zumindest zwei Anschlüsse mit der Versorgungsvorrichtung verbunden und von der Versorgungsvorrichtung mit einem Reinigungs- oder Pflegemittel versorgt wird, während ein anderer der zumindest zwei Anschlüsse nicht mit der Versorgungsvorrichtung verbunden wird und kein Reinigungs- oder Pflegemittel von der Versorgungsvorrichtung erhält. Insbesondere werden die zumindest zwei Anschlüsse nacheinander mit der Versorgungsvorrichtung verbunden und von der Versorgungsvorrichtung mit einem Reinigungs- oder Pflegemittel versorgt, so dass zumindest ein Teil des Reinigungs- oder Pflegeverfahrens, insbesondere die Reinigung oder Pflege des Inneren der medizinischen, insbesondere dentalen, Instrumente, sequentiell abläuft. Das Verfahren umfasst bevorzugt des Weiteren das Merkmal, dass nur jene der zumindest zwei Anschlüsse mit der Versorgungsvorrichtung verbunden und von der Versorgungsvorrichtung mit einem Reinigungs- oder Pflegemittel versorgt werden, die mit einem medizinischen, insbesondere dentalen, Instrument belegt sind.

Gemäß einem weiteren Ausführungsbeispiel ist vorgesehen, das Reinigungs- oder Pflegeverfahren derart zu gestalten, dass zuerst die Innenreinigung oder -pflege aller Instrumente erfolgt und anschließend die Außenreinigung oder -pflege der Instrumente durchgeführt wird. Damit wird die Gefahr der gegenseitigen Kontamination der Instrumente verringert, insbesondere die Kontamination eines außen bereits gereinigten Instruments durch kontaminierte Reinigungsflüssigkeit, die aus dem Inneren eines anderen Instruments spritzt. Bevorzugt sind die Düsen für die Außenreinigung oder -pflege derart im Reinigungs- oder Pflegegerät angeordnet, dass im Wesentlichen nur ein Anschluss oder ein damit verbundenes Instrument mit Reinigungs- oder Pflegemittel beaufschlagt wird, während zumindest ein zweiter Anschluss oder ein zweites damit verbundenes Instrument im Wesentlichen nicht mit Reinigungs- und/oder Pflegemittel beaufschlagt wird. Ein Reinigungs- oder Pflegeverfahren, das eine Innen- und Außenreinigung oder -pflege beinhaltet, würde demgemäß derart ablaufen, dass für die Innenreinigung oder -pflege zuerst nacheinander jeder der zumindest zwei Anschlüsse mit der Versorgungsvorrichtung verbunden wird, um Reinigungs- oder Pflegemittel in das Innere der Instrumente zu leiten, und anschließend nacheinander jeder der zumindest zwei Anschlüsse derart in Bezug auf die Düsen positioniert wird, dass die Außenreinigung oder -pflege der einzelnen Instrumente erfolgt. Alternativ ist es natürlich auch möglich, für die Außenreinigung die Düsen sequentiell zu den jeweiligen Instrumenten oder relativ zu den zumindest zwei Anschlüssen zu bewegen.

Die Erfindung wird nachfolgend anhand bevorzugter Ausführungsbeispiele und Bezug nehmend auf die beigefügten Zeichnungen erläutert:
Figur 1 zeigt eine Außenansicht eines Ausführungsbeispiels eines Reinigungs- oder Pflegegeräts, bei dem die Anschlüsse für die zu reinigenden Instrumente und die Versorgungsvorrichtung relativ zueinander bewegbar sind.
Figur 2 zeigt eine Schnittdarstellung durch das Reinigungs- oder Pflegegerät der Figur 1.
Figur 3 zeigt in einer perspektivischen Darstellung ein Ausführungsbeispiel eines relativ zur Versorgungsvorrichtung bewegbaren, insbesondere drehbaren, Basiselements mit einem Verteilerelement für ein Reinigungs- oder Pflegemittel und einem Antriebsmotor.
Figur 4 zeigt eine Schnittdarstellung durch das Basiselement, das Verteilerelement und den Antriebsmotor der Figur 3.
Figur 5 zeigt eine Schnittdarstellung durch das Basiselement der Figur 4.
Figur 6 zeigt eine Aufsicht auf die der Versorgungsvorrichtung zugewandte Rückseite des von dem Reinigungs- oder Pflegegerät getrennten Basiselements der Figur 5 einschließlich des ersten Verteilerstücks des Verteilerelements.

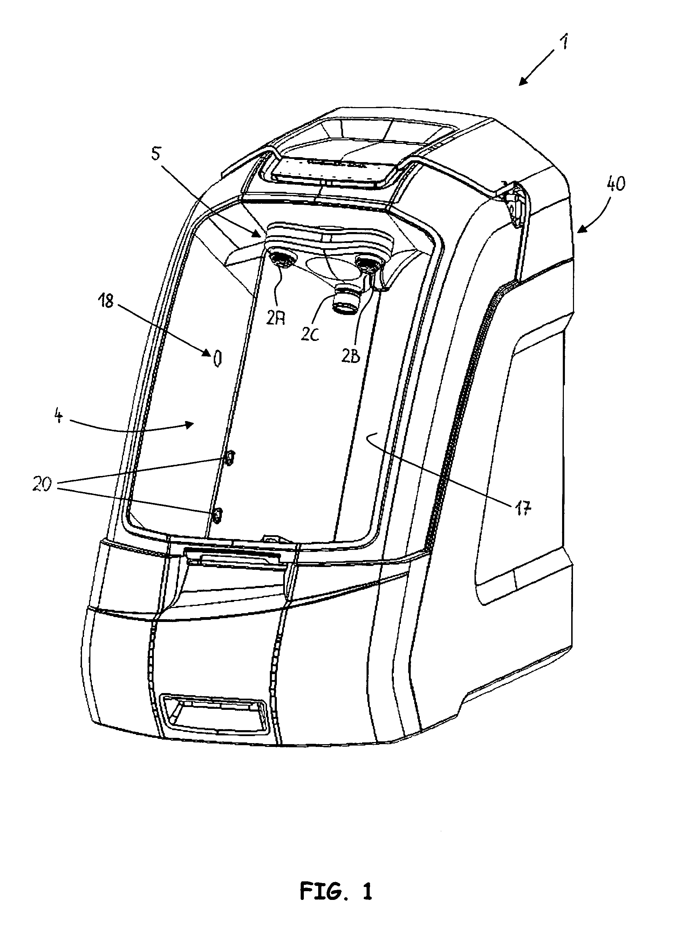

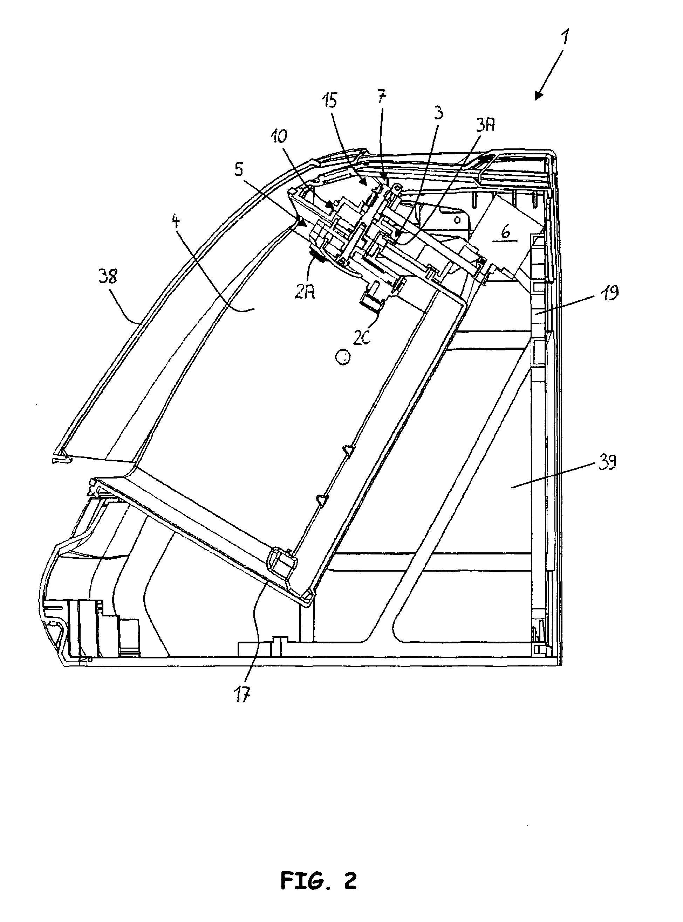

Das in den Figuren 1 und 2 dargestellte Reinigungs- oder Pflegegerät 1 umfasst ein, bevorzugt aus Kunststoff hergestelltes, Außengehäuse 40 mit einer Bodenplatte, mehreren Seitenwänden und einer Oberplatte. Eine der Seitenwände weist eine bewegliche, insbesondere verschwenkbare, Türe oder einen beweglichen, insbesondere verschwenkbaren, Deckel 38 auf, der bevorzugt über Scharniere an der Oberplatte bewegbar ist. Mit dem Deckel 38 ist ein im Inneren des Gehäuses 40 angeordnete Reinigungs- oder Pflegekammer 4 verschließbar, in dem mehrere Kupplungen oder Anschlüsse, im vorliegenden Fall drei Anschlüsse 2A, 2B, 2C, für zu reinigende oder zu pflegende medizinische, insbesondere dentale, Instrumente vorgesehen sind. Mittels der Anschlüsse 2A, 2B, 2C werden ein oder mehrere Reinigungs- oder Pflegemittel in das Innere der Instrumente gefördert. Zusätzlich befinden sich in der Reinigungs- oder Pflegekammer 4 eine oder mehrere Öffnungen oder Düsen 20, über die ein Reinigungs- oder Pflegemittel auf die Außenseite der Instrumente abgegeben werden kann. Die Düsen 20 sind von den Anschlüssen 2A, 2B, 2C getrennt angeordnet und, insbesondere zeitlich, unabhängig von den Düsen 2A, 2B, 2C mit einem Reinigungs- und/oder Pflegemittel versorgbar.

Die Reinigungs- oder Pflegekammer 4 wird durch eine im Inneren des Reinigungs- oder Pflegegeräts 1 angeordnete Trennwand 17 von einem Steuerraum 39 (siehe Figur 2) getrennt. Am oder im Steuerraum 39 sind eine Vielzahl von Steuer- und Funktionselementen des Reinigungs- oder Pflegegeräts 1 vorgesehen, zum Beispiel eine Versorgungsvorrichtung 3, von der in der Figur 2 aus Gründen der Übersichtlichkeit nur ein kurzes Leitungsstück 3A dargestellt ist und die unter anderem Behälter für Reinigungs-, Pflege- oder Schmiermittel, Anschlüsse an eine oder mehrere Fluidquellen, zum Beispiel an eine Druckluft- und oder Wasserquelle, Leitungen zur Förderung der Fluide, Reinigungs-, Pflege- oder Schmiermittel, Pumpen, Steuer- und Stellmittel, zum Beispiel Ventile, Drosseln, Sensoren, zum Beispiel zur Durchflussmessung, Konzentrationsmessung, Mischer und viele weitere Bauteile enthalten kann. Neben der Versorgungsvorrichtung 3 können am oder im Steuerraum 39 weitere Bauteile, wie zum Beispiel ein Anschluss an eine Energiequelle, Auffangwannen oder Ablässe für gebrauchte Reinigungs- oder Pflegemittel, eine Steuervorrichtung 19 zur Steuerung und / oder Regelung der Reinigungs- oder Pflegeverfahren, eine Speichereinheit, eine Detektionseinheit 15 zur Positionierung der Anschlüsse 2A, 2B, 2C in einer vorbestimmten Startposition, eine Betriebsanzeige oder ein Bedienpanel für den Anwender vorgesehen sein.

Eine in Figur 1 zu erkennende Detektionseinheit 18 zur Bestimmung welcher der Anschlüsse 2A, 2B, 2C mit einem medizinischen, insbesondere dentalen, Instrument belegt ist und / oder welcher der Anschlüsse 2A, 2B, 2C nicht mit einem Instrument belegt ist umfasst bevorzugt eine Strahlungsquelle, insbesondere zur Abgabe von Infrarotstrahlung, und einen Sensor, insbesondere einen Infrarotsensor, zur Detektion der Strahlung. Die Strahlungsquelle und der Sensor sind an unterschiedlichen Abschnitten der Trennwand 17 vorgesehen. Die Strahlungsquelle emittiert ihre Strahlung durch die Reinigungs- oder Pflegekammer 4 in Richtung des Sensors. Ist ein Instrument an einen der Anschlüsse 2A, 2B, 2C gekuppelt, so wird die Strahlung durch das Instrument abgeschwächt oder unterbrochen, wodurch der Sensor das Vorhandensein eines Instruments erkennt. Der Sensor erzeugt entsprechende Belegungssignale und gibt diese an die Steuervorrichtung 19 ab, die auf Basis dieser Belegungssignale nur jene Anschlüsse 2A, 2B, 2C mit Reinigungs- oder Pflegemittel versorgt, an denen ein medizinisches, insbesondere dentales, Instrument angeschlossen ist.

Die Anschlüsse 2A, 2B, 2C und die Versorgungsvorrichtung 3 sind derart relativ zueinander bewegbar, dass zumindest einer der Anschlüsse 2A, 2B, 2C mit der Versorgungsvorrichtung 3 verbunden und mit einem Reinigungs- oder Pflegemittel versorgbar ist, während zumindest ein anderer der Anschlüsse 2A, 2B, 2C nicht mit der Versorgungsvorrichtung 3 verbunden ist und nicht mit einem Reinigungs- oder Pflegemittel versorgbar ist. Dazu sind die Anschlüsse 2A, 2B, 2C auf einem relativ zur Versorgungsvorrichtung 3 beweglichen Basiselement 5 angeordnet, das insbesondere als relativ zur Versorgungsvorrichtung 3 drehbares Drehelement ausgebildet ist. Das Basiselement 5 wird über einen im Steuerraum 39 angeordneten Antriebsmotor 6, der bevorzugt als Schrittmotor ausgebildet ist, und ein oder mehrere Antriebselemente 7 in Drehung versetzt. Der Betrieb des Antriebsmotors 6, zum Beispiel die Steuerung der Schrittanzahl des Schrittmotors, erfolgt über die Steuervorrichtung 19 und in Abhängigkeit des von der Steuervorrichtung 19 ausgeführten Reinigungs- oder Pflegeprogramms. Somit ist das Basiselement 5 relativ zur Versorgungsvorrichtung 3 bewegbar (drehbar) und derart positionierbar, dass wahlweise einer der Anschlüsse 2A, 2B, 2C mit der Versorgungsvorrichtung 3 verbunden und somit mit Reinigungs- oder Pflegemittel versorgbar ist, während ein anderer der Anschlüsse 2A, 2B, 2C nicht mit der Versorgungsvorrichtung verbunden und damit nicht mit Reinigungs- oder Pflegemittel versorgbar ist.

Die Versorgung der Anschlüsse 2A, 2B, 2C mit Reinigungs- oder Pflegemitteln erfolgt über ein Verteilerelement 10, das zwischen dem Basiselement 5 und der Versorgungsvorrichtung 3 angeordnet ist.

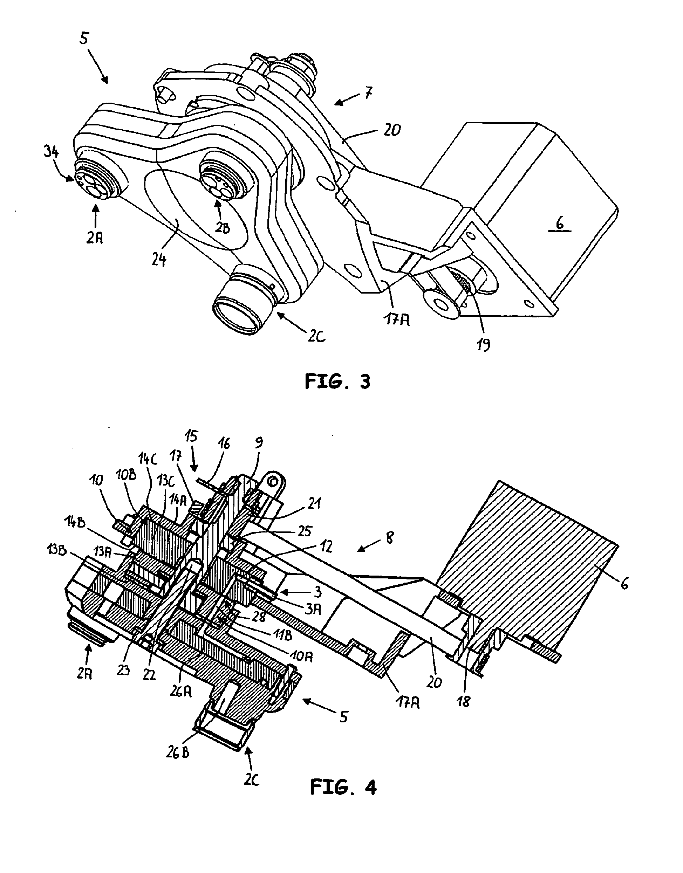

In den Figuren 3 und 4 sind das Basiselement 5 mit den Anschlüssen 2A, 2B, 2C, der Antriebsmotor 6 mit den Antriebselementen 7, die Detektionseinheit 15 zur Positionierung des Basiselements 5 oder der Anschlüsse 2A, 2B, 2C und das Verteilerelement 10 im Detail dargestellt. Als Aufhängung insbesondere für den Antriebsmotor 6, die Antriebselemente 7 und das Basiselement 5 dient eine Wand 17A, in der Gewindebohrungen vorgesehen sind, so dass die oben genannten Elemente mittels Gewindeschrauben an der Trennwand verschraubt werden können.

Der Antrieb des Basis- oder Drehelements 5 erfolgt durch den Schrittmotor 6 und einen Riemenantrieb 8. Auf der Rotorwelle 18 des Schrittmotors 6 ist eine erste, insbesondere gezahnte, Riemenscheibe 19 befestigt. Ein, bevorzugt als Zahnriemen ausgebildeter, Riemen 20 überträgt die Antriebsbewegung des Schrittmotors 6 von der ersten Riemenscheibe 19 auf eine zweite, insbesondere gezahnte, Riemenscheibe 21, die mit einer Welle 9 verbunden ist. Die Welle 9 ist an zwei Lagerstellen gelagert, wobei zum Beispiel gemäß der Figur 4 eine Lagerstelle durch die Trennwand 17 und die andere Lagerstelle durch das Verteilerelement 10 gebildet ist. An der durch die Wand 17A gebildeten Lagerstelle ist bevorzugt ein Wälzlager 25 vorgesehen. Die Welle 9 ragt mit einem Ende durch eine Bohrung in der Trennwand 17 in die Reinigungs- oder Pflegekammer 4. An diesem Ende der Welle 9 ist eine Gewindebohrung 22 vorgesehen, in die zur Verbindung der Welle 9 mit dem Drehelement 5 und den Anschlüssen 2A, 2B, 2C ein das Drehelement 5 durchsetzender Gewindestift 23 einfügbar ist. Die Gewindebohrung 22 und der Gewindestift 23 werden durch eine am Drehelement 5 befestigbare Kappe 24 abgedeckt.

Das Verteilerelement 10 zur Versorgung der Anschlüsse 2A, 2B, 2C mit Reinigungs- oder Pflegemitteln ist zweiteilig aufgebaut. Es besteht aus einem ersten, mit dem Basiselement 5 relativ zur Versorgungsvorrichtung 3 bewegbaren Verteilerstück 10A und einem zweiten, relativ zur Versorgungsvorrichtung 3 festen oder unbeweglichen Verteilerstück 10B. Beide Verteilerstücke 10A, 10B weisen eine in etwa mittige Bohrung zur Aufnahme der Welle 9 auf. In der Bohrung des Verteilerstücks 10B ist des Weiteren ein Lager, insbesondere ein Gleitlager, für die Welle 9 vorgesehen.

Das zweite Verteilerstück 10B ist in einem Rücksprung der Wand 17A aufgenommen oder befestigt. Es weist zumindest eine, bevorzugt mehrere, Leitungen 12 für Reinigungs- oder Pflegemittel auf, die mit der Versorgungsvorrichtung 3 verbunden sind, so dass die Reinigungs- oder Pflegemittel von der Versorgungsvorrichtung 3 in die Leitungen 12 übertreten können. Beispielhaft ist in der Figur 4 zu erkennen, wie die Leitung 3A der Versorgungsvorrichtung 3 durch die Wand 17A tritt, um zu der an einer Oberfläche des zweiten Verteilerstücks 10B gelegenen Aufnahmeöffnung der Leitung 12 zu gelangen. Die Leitung 12 durchsetzt das zweite Verteilerstück 10B und bildet an einer Oberfläche des zweiten Verteilerstücks 10B eine dem ersten Verteilerstück 10A zugewandte Abgabeöffnung, über die die Reinigungs- oder Pflegemittel vom zweiten Verteilerstück 10B auf das erste Verteilerstück 10A abgegeben werden können. Die Abgabeöffnung der einen Leitung oder die Abgabeöffnungen der mehreren Leitungen 12 definieren an der Oberfläche des zweiten Verteilerstücks 10B eine einzige Schnitt- oder Übergabestelle 28.

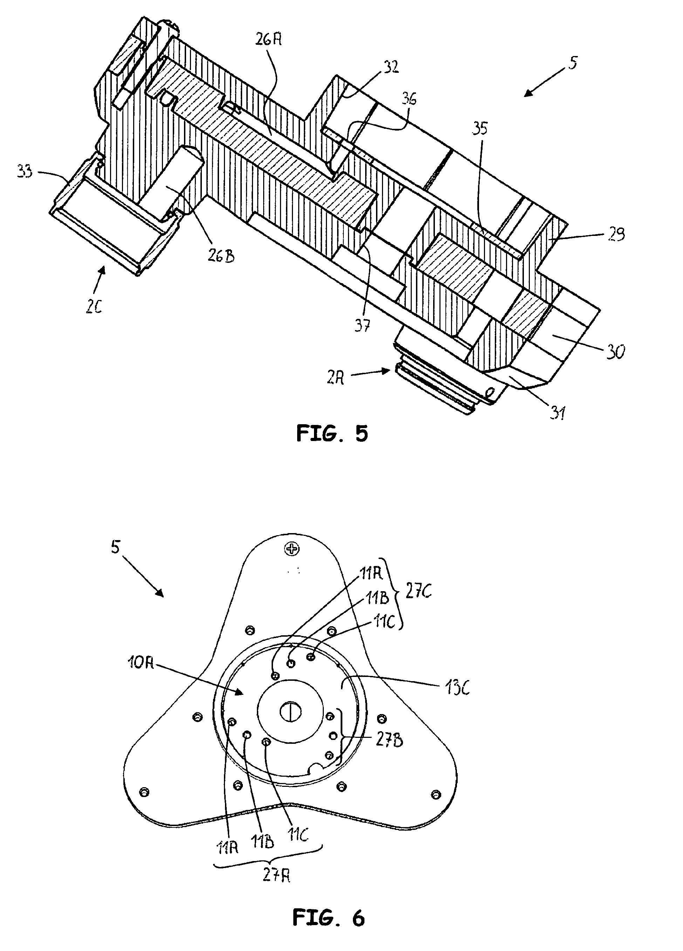

Das erste Verteilerstück 10A ist in einer Aufnahme 32 (siehe Figur 5) des Basiselements 5 aufgenommen. Es weist zumindest jeweils eine eigene Leitung für jeden der Anschlüsse 2A, 2B, 2C auf. Gemäß dem Ausführungsbeispiel nach Figur 6 sind im ersten Verteilerstück 10A für jeden Anschluss 2A, 2B, 2C drei eigene Leitung 11A, 11B, 11C vorgesehen, wobei an jeden Anschluss 2A, 2B, 2C jeweils durch die Leitung 11A ein Reinigungsmittel, durch die Leitung 11B Druckluft und durch die Leitung 11C ein Schmiermittel gefördert werden. Jede der Leitungen 11A, 11B, 11C durchsetzt das erste Verteilerstück 10A und weist somit an den Oberflächen des ersten Verteilerstücks 10A jeweils eine Aufnahmeöffnung für die Reinigungs- oder Pflegemittel, die dem zweiten Verteilerstück 10B zugewandt ist, und eine Abgabeöffnung für die Reinigungs- oder Pflegemittel, die den Anschlüssen 2A, 2B, 2C zugewandt ist, auf. Von den Abgabeöffnungen gelangen die Reinigungs- oder Pflegemittel zu den jeweiligen Anschlüssen 2A, 2B, 2C, insbesondere durch Bohrungen oder Leitungen 26A, 26B im Basiselement 5. Die zumindest eine Leitung oder die mehreren Leitungen 11A, 11B, 11C, die jeweils einem der Anschlüsse 2A, 2B, 2C zugeordnet sind, definieren an ihren Aufnahmeöffnungen eine weitere Schnitt-oder Übergabestelle. Gemäß dem Ausführungsbeispiel der Figur 6 gibt es somit drei Übergabestellen 27A, 27B, 27C für die drei Anschlüsse 2A, 2B, 2C, wobei jede Übergabestelle 27A die drei Aufnahmeöffnungen der drei Leitungen 11A, 11B, 11C eines Anschlusses 2A, 2B, 2C umfasst.

Durch das Bewegen, insbesondere Drehen, des ersten, mit dem Basiselement 5 verbundenen Verteilerstücks 10A relativ zum zweiten, in Bezug auf das Basiselement 5 oder die Anschlüsse 2A, 2B, 2C drehfesten Verteilerstück 10B sind die Aufnahmeöffnungen und Leitungen 11A, 11B, 11C des ersten Verteilerstücks 10A mit der zumindest einen Leitung 12 und deren Abgabeöffnung des zweiten Verteilerstücks 10B verbindbar. Insbesondere wird die zumindest eine Leitung oder werden die mehreren Leitungen 11A, 11B, 11C eines Anschlusses 2A, 2B, 2C mit der zumindest einen Leitung 12 des zweiten Verteilerstück 10B verbunden oder gekuppelt, so dass dieser Anschluss 2A, 2B, 2C mit einem Reinigungs- oder Pflegemittel versorgbar ist, während die Leitungen 11A, 11B, 11C der anderen Anschlüsse 2A, 2B, 2C nicht mit der zumindest einen Leitung 12 des zweiten Verteilerstück 10B verbunden oder gekuppelt sind und somit nicht mit einem Reinigungs- oder Pflegemittel versorgbar sind. Gemäß dem Ausführungsbeispiel der Figur 6 ist eine der drei Übergabestellen 27A, 27B, 27C des ersten Verteilerstücks 10A mit der einzigen Übergabestelle 28 des zweiten Verteilerstücks 10B verbunden oder überlagern sich diese beiden Übergabestellen, während gleichzeitig die anderen beiden Übergabestellen 27A, 27B, 27C des ersten Verteilerstücks 10A nicht mit der Übergabestelle 28 des zweiten Verteilerstücks 10B verbunden sind. Damit ist jeweils nur eine der drei Anschlüsse 2A, 2B, 2C mit einem Reinigungs- oder Pflegemittel versorgbar, wobei durch Verdrehen des Basiselements 5 jeder der drei Anschlüsse 2A, 2B, 2C zeitlich hintereinander mit der Übergabestelle 28 verbunden werden kann und somit eine sequentielle Versorgung der drei Anschlüsse 2A, 2B, 2C während eines Reinigungs- oder Pflegezykluses möglich wird.

Wie aus den Figuren 4 und 6 des Weiteren zu erkennen ist, sind das erste Verteilerstück 10A und das zweite Verteilerstück 10B scheibenförmig geformt, wobei jedes Verteilerstück 10A, 10B eine Außenmantelfläche 13A, 14A und zwei Deckflächen 13B, 13C, 14B, 14C aufweist. Die beiden Verteilerstücke 10A, 10B sind mit jeweils einer Deckfläche 13C, 14B aneinander gleitend angeordnet An diesen beiden Deckflächen 13C, 14B sind demgemäß auch die Übergabestellen 27A, 27B, 27C, 28 mit ihren jeweiligen Leitungen oder Öffnungen vorgesehen. Die beiden Deckflächen 13C, 14B bilden somit auch ein Gleitlager, an dem das Drehelement drehbar gelagert ist.

Die Detektionseinheit 15 zur Positionierung des Basiselements 5 in einer vorbestimmten Startposition umfasst eine Markierung 16 an einem der Antriebselemente 7 und einen die Markierung 16 erkennenden Sensor 17. Gemäß dem in der Figur 4 dargestellten Ausführungsbeispiel weist die Detektionseinheit 15 zusätzlich eine (nicht dargestellte) Strahlungs- oder Lichtquelle auf, die Strahlung in Richtung des als optischen Sensor ausgebildeten Sensors 17 emittiert, wobei die Markierung 16 zwischen dem optischen Sensor 17 und der Strahlungs- oder Lichtquelle angeordnet ist. Die Markierung 16 ist als Fortsatz oder Stift ausgebildet, der unbeweglich an der Welle 9 befestigt ist und sich mit der Welle 9 dreht. Die Startposition ist als jene Position definiert, in der die Markierung 16 derart angeordnet ist, dass sie den von der Strahlungs- oder Lichtquelle emittierten Strahl abschwächt oder unterbricht, so dass die zum Sensor 17 gelangende Strahlung zumindest deutlich verringert ist. Befindet sich die Markierung 16 in der Startposition, so nehmen die ebenfalls fix mit der Welle 9 verbundenen Anschlüsse 2A, 2B, 2C eine vorbestimmte Position ein.

Der Sensor 17 ist mit der Steuervorrichtung 19 verbunden und sendet Positionssignale, die die Position der Markierung 16 und damit der Anschlüsse 2A, 2B, 2C wiedergibt. Die Steuervorrichtung 19 ist derart ausgebildet, dass sie den Antriebsmotor 6 und die Welle 9 so lange antreibt, bis die Startposition erreicht ist. Bei Erreichen der Startposition stoppt die Steuervorrichtung 19 den Antriebsmotor 6, so dass die Anschlüsse 2A, 2B, 2C ihre vorbestimmte Position einnehmen. Ausgehend von dieser Position ist sichergestellt, dass jeder der Anschlüsse 2A, 2B, 2C zuverlässig mit der Versorgungsvorrichtung 3 verbindbar ist, in dem die Steuervorrichtung 19 dem Schrittmotor 6 eine vorbestimmten Schrittfolge oder Schrittzahl vorgibt. Die Einnahme der Startposition erfolgt bevorzugt vor Beginn der Abgabe von Reinigungs- oder Pflegemittel an die Anschlüsse 2A, 2B, 2C.

Der Aufbau des Basiselements 5 ist insbesondere aus den Figuren 5 und 6 ersichtlich. Das Basiselements 5 besteht aus drei Lagen oder Schichten: Einem, bevorzugt aus Kunststoff hergestellten, der Trennwand 17 nächstgelegenem Rückteil 29, einem daran anschließenden, bevorzugt aus einem Elastomer hergestellten, Dichtteil 30 und einem daran anschließenden Vorderteil 31, das bevorzugt wiederum aus Kunststoff hergestellt ist. Die drei Teile 29, 30, 31 werden durch Gewindestifte, die in entsprechenden Gewindebohrungen der Teile 29, 30, 31 eingeschraubt sind, miteinander verbunden. Die das Basiselement 5 durchsetzende Bohrung 37 dient zur Aufnahme des Gewindestifts 23.

Am Rückteil 29 ist die kreisförmige Aufnahme 32 für das erste Verteilerstück 10A vorgesehen. In der Aufnahme 32 befindet sich zusätzlich eine Dichtscheibe 35. Am Vorderteil 31 sind die drei Anschlüsse 2A, 2B, 2C angeordnet, wobei bevorzugt zumindest Teile der Anschlüsse 2A, 2B, 2C integral mit dem Vorderteil 31 ausgebildet sind. Am Anschluss 2C ist des Weiteren eine metallische Kupplungshülse 33 für die an die Anschlüsse 2A, 2B, 2C zu kuppelnden Instrumente zu erkennen.

Alle drei Teile 29, 30, 31 des Basiselements 5 sind von Bohrungen oder Leitungen 26A, 26B zur Leitung der Reinigungs- oder Pflegemittel zu den Anschlüssen 2A, 2B, 2C durchsetzt, wobei jedem Anschluss 2A, 2B, 2C zumindest eine derartige Leitung 26A, 26B zugeordnet ist. Diese Leitungen 26A, 26B enden in den Öffnungen 34 der Anschlüssen 2A, 2B, 2C, die in der Figur 3 zu erkennen sind. An diese Öffnungen sind die Medienleitungen der zu reinigenden oder zu pflegenden Instrumente ankuppelbar, so dass die Reinigungs- oder Pflegemittel in das Innere der Instrumente, insbesondere in ihre Medienleitungen gelangen können. Die Leitungen 26A, 26B enden im Rückteil 29 in Öffnungen (beispielhaft ist in Figur 5 eine Öffnung 36 zu erkennen), die an die Leitungen 11A, 11B, 11C des ersten Verteilerstücks 10A anschließen, um die Reinigungs- oder Pflegemittel vom ersten Verteilerstück 10A zu übernehmen.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Reinigungs- oder Pflegegerät (1) zur Reinigung oder Pflege medizinischer, insbesondere dentaler, Instrumente, umfassend eine Reinigungs- oder Pflegekammer (4), in der zumindest zwei Anschlüsse (2A, 2B, 2C) für zu reinigende Instrumente vorgesehen sind, und eine Versorgungsvorrichtung (3) zur Versorgung der zumindest zwei Anschlüsse (2A, 2B, 2C) mit einem Reinigungs- oder Pflegemittel, wobei
die Versorgungsvorrichtung (3) eine Übergabestelle zur wahlweisen Verbindung mit einem der zumindest zwei Anschlüsse (2A, 2B, 2C) aufweist, an welcher das oder die Reinigungs- oder Pflegemittel übergeben werden, wobei an dieser Übergabestelle mehrere Leitungen (26A, 26B) für Reinigungs- oder Pflegemittel vorgesehen sind, wobei diese Leitungen (26A, 26B) in Öffnungen (34) der zumindest zwei Anschlüsse (2A, 2B, 2C) enden, an welche Medienleitungen der medizinischen, insbesondere dentalen, Instrumente ankuppelbar sind, so dass die Reinigungs- oder Pflegemittel in das Innere der medizinischen, insbesondere dentalen, Instrumente förderbar sind, **dadurch gekennzeichnet, dass** die zumindest zwei Anschlüsse (2A, 2B, 2C) und die Versorgungsvorrichtung (3) derart relativ zueinander bewegbar sind, dass einer der zumindest zwei Anschlüsse (2A, 2B, 2C) mit der Versorgungsvorrichtung (3) verbunden und mit einem Reinigungs- oder Pflegemittel versorgbar ist, während ein anderer der zumindest zwei Anschlüsse (2A, 2B, 2C) nicht mit der Versorgungsvorrichtung (3) verbunden ist und nicht mit einem Reinigungs- oder Pflegemittel versorgbar ist.

2. Reinigungs- oder Pflegegerät (1) nach Anspruch 1, **dadurch gekennzeichnet, dass** die zumindest zwei Anschlüsse (2A, 2B, 2C) auf einem gemeinsamen Basiselement (5) angeordnet sind, das derart relativ zur Versorgungsvorrichtung (3) bewegbar ist, dass einer der zumindest zwei Anschlüsse (2A, 2B, 2C) mit der Versorgungsvorrichtung (3) verbunden ist, während ein anderer der zumindest zwei Anschlüsse (2A, 2B, 2C) nicht mit der Versorgungsvorrichtung (3) verbunden ist.

3. Reinigungs- oder Pflegegerät (1) nach Anspruch 1 oder 2, **gekennzeichnet durch** einen außerhalb der Reinigungs- oder Pflegekammer (4) angeordneten Antriebsmotor (6), zum Beispiel einen Elektromotor, insbesondere einen Schrittmotor, zur Bewegung der zumindest zwei Anschlüsse (2A, 2B, 2C) und der Versorgungsvorrichtung (3) relativ zueinander, insbesondere zum Antrieb des Basiselements (5).

4. Reinigungs- oder Pflegegerät (1) nach Anspruch 3, **dadurch gekennzeichnet, dass** der Antriebsmotor (6) über zumindest ein Antriebselement (7), zum Beispiel einen Riemenantrieb (8) und / oder eine Welle (9), mit den zumindest zwei Anschlüssen (2A, 2B, 2C) und / oder der Versorgungsvorrichtung (3) und / oder dem Basiselement (5) verbunden ist.

5. Reinigungs- oder Pflegegerät (1) nach einem der Ansprüche 2 - 4, **gekennzeichnet durch**
ein zwischen dem Basiselement (5) und der Versorgungsvorrichtung (3) vorgesehenes Verteilerelement (10) zum Leiten eines Reinigungs- oder Pflegemittels von der Versorgungsvorrichtung (3) zu den zumindest zwei Anschlüssen (2A, 2B, 2C), wobei das Verteilerelement (10) ein erstes, mit dem Basiselement (5) relativ zur Versorgungsvorrichtung (3) bewegbares Verteilerstück (10A) und ein zweites, relativ zur Versorgungsvorrichtung (3) unbewegliches Verteilerstück (10B) aufweist.

6. Reinigungs- oder Pflegegerät (1) nach Anspruch 5, **dadurch gekennzeichnet, dass** das erste Verteilerstück (10A) zumindest jeweils eine eigene Leitung (11A, 11 B, 11 C) für jeden der zumindest zwei Anschlüsse (2A, 2B, 2C) aufweist.

7. Reinigungs- oder Pflegegerät (1) nach Anspruch 6, **dadurch gekennzeichnet, dass** das zweite Verteilerstück (10B) zumindest eine Leitung (12) für ein Reinigungs- oder Pflegemittel aufweist, wobei durch das Bewegen des ersten Verteilerstücks (10A) relativ zum zweiten Verteilerstück (10B) die Leitungen (11 A, 11 B, 11 C) des ersten Verteilerstücks (10A) mit der zumindest einen Leitung (12) des zweiten Verteilerstücks (10B) verbindbar sind.

8. Reinigungs- oder Pflegegerät (1) nach einem der Ansprüche 5 - 7, **gekennzeichnet durch**
scheibenförmige erste und das zweite Verteilerstücke (10A, 10B), wobei jedes Verteilerstück (10A, 10B) eine Außenmantelfläche (13A, 14A) und zwei Deckflächen (13B, 13C; 14B, 14C) aufweist und wobei die beiden Verteilerstücke (10A, 10B) mit jeweils einer Deckfläche (13C, 14B) aneinander gleitend angeordnet sind.

9. Reinigungs- oder Pflegegerät (1) nach einem der Ansprüche 2 - 8, **gekennzeichnet durch**
eine Detektionseinheit (15) zur Positionierung des Basiselements (5) in einer vorbestimmten Startposition.

10. Reinigungs- oder Pflegegerät (1) nach Anspruch 9, **dadurch gekennzeichnet, dass** die Detektionseinheit (15) eine Markierung (16) an einem den Antriebsmotor (6) mit dem Basiselement (5) verbindenden Antriebselement (7) und einen die Markierung (16) erkennenden Sensor (17) umfasst.

11. Reinigungs- oder Pflegegerät (1) nach einem der vorherstehenden Ansprüche, **gekennzeichnet durch**
eine Detektionseinheit (18) zur Bestimmung welcher der zumindest zwei Anschlüsse (2A, 2B, 2C) mit einem medizinischen, insbesondere dentalen, Instrument belegt ist und / oder welcher der zumindest zwei Anschlüsse (2A, 2B, 2C) nicht mit einem medizinischen, insbesondere dentalen, Instrument belegt ist und zur Abgabe entsprechender Belegungssignale, wobei die Detektionseinheit (18) mit einer Steuervorrichtung (19) verbunden ist, die auf Basis der von der Detektionseinheit (18) abgegebenen Belegungssignale ausgebildet ist, nur jene der zumindest zwei Anschlüsse (2A, 2B, 2C) mit Reinigungs- oder Pflegemittel zu versorgen, an denen ein medizinisches, insbesondere dentales, Instrument angeschlossen ist.

12. Reinigungs- oder Pflegegerät (1) nach einem der vorherstehenden Ansprüche, **dadurch gekennzeichnet, dass**
von den zumindest zwei Anschlüssen (2A, 2B, 2C) getrennt angeordnete Düsen (20) zur Abgabe eines Reinigungs- oder Pflegemittels auf die Außenseite der medizinischen, insbesondere dentalen, Instrumente vorgesehen sind.

13. Verfahren zur Reinigung oder Pflege medizinischer, insbesondere dentaler, Instrumente, mit einem Reinigungs- oder Pflegegerät (1) nach einem der vorherstehenden Ansprüche, **dadurch gekennzeichnet, dass**
einer der zumindest zwei Anschlüsse (2A, 2B, 2C) mit der Versorgungsvorrichtung (3) verbunden und von der Versorgungsvorrichtung (3) mit einem Reinigungs- oder Pflegemittel versorgt wird, während ein anderer der zumindest zwei Anschlüsse (2A, 2B, 2C) nicht mit der Versorgungsvorrichtung (3) verbunden wird und kein Reinigungs- oder Pflegemittel von der Versorgungsvorrichtung (3) erhält.

14. Verfahren zur Reinigung oder Pflege medizinischer, insbesondere dentaler, Instrumente nach Anspruch 13, **dadurch gekennzeichnet, dass** die zumindest zwei Anschlüsse (2A, 2B, 2C) nacheinander mit der Versorgungsvorrichtung (3) verbunden und von der Versorgungsvorrichtung (3) mit einem Reinigungs- oder Pflegemittel versorgt werden, so dass zumindest ein Teil des Reinigungs- oder Pflegeverfahrens, insbesondere die Reinigung oder Pflege des Inneren der medizinischen, insbesondere dentalen, Instrumente, sequentiell abläuft.

15. Verfahren zur Reinigung oder Pflege medizinischer, insbesondere dentaler, Instrumente nach Anspruch 13 oder 14, **dadurch gekennzeichnet, dass** nur jene der zumindest zwei Anschlüsse (2A, 2B, 2C) mit der Versorgungsvorrichtung (3) verbunden und von der Versorgungsvorrichtung (3) mit einem Reinigungs- oder Pflegemittel versorgt werden, die mit einem medizinischen, insbesondere dentalen, Instrument belegt sind.

## Claims

1. A cleaning or care device (1) for cleaning or care of medical, in particular dental, instruments, comprising a cleaning or care chamber (4), in which at least two connections (2A, 2B, 2C) for instruments to be cleaned are provided, and a supply device (3) for supplying the at least two connections (2A, 2B, 2C) with a cleaning or care agent, wherein the supply device (3) has a transfer location for optional connection to one of the at least two connections (2A, 2B, 2C), at which the cleaning or care agent(s) is/are transferred, wherein several lines (26A, 26B) for cleaning or care agents are provided at this transfer location, wherein these lines (26A, 26B) end in openings (34) of the at least two connections (2A, 2B, 2C) to which media lines of the medical, in particular, dental, instruments can be connected, so that the cleaning or care agents can be conveyed into the interior of the medical, in particular dental, instruments, **characterized in that**
the at least two connections (2A, 2B, 2C) and the supply device (3) can be moved in relation to one another, such that one of the at least two connections (2A, 2B, 2C) is connected to the supply device (3) and can be supplied with a cleaning or care agent, while another of the at least two connections (2A, 2B, 2C) is not connected to the supply device (3) and cannot be supplied with a cleaning or care agent.

2. The cleaning or care device (1) according to Claim 1, **characterized in that** the at least two connections (2A, 2B, 2C) are arranged on a shared base element (5), which can be moved in relation to the supply device (3), such that one of the at least two connections (2A, 2B, 2C) is connected to the supply device (3), while another of the at least two connections (2A, 2B, 2C) is not connected to the supply device (3).

3. The cleaning or care device (1) according to Claim 1 or 2, **characterized by** a drive motor (6), for example an electric motor, in particular a stepping motor, which is arranged outside of the cleaning or care chamber (4), for moving the at least two connections (2A, 2B, 2C) and the supply device (3) in relation to one another, in particular for driving the base element (5).

4. The cleaning or care device according to Claim 3, **characterized in that** the drive motor (6) is connected via at least one drive element (7), for example, a belt drive (8) and/or a shaft (9) to the at least two connections (2A, 2B, 2C) and/or the supply device (3) and/or the base element (5).

5. The cleaning or care device (1) according to any one of Claims 2 - 4, **characterized by** a distributor element (10), which is provided between the base element (5) and the supply device (3) for conducting a cleaning or care agent from the supply device (3) to the at least two connections (2A, 2B, 2C), wherein the distributor element (10) has a first distributor piece (10A), which is movable with the base element (5) in relation to the supply device (3) and has a second distributor piece (10B) which is immovable in relation to the supply device (3).

6. The cleaning or care device (1) according to Claim 5, **characterized in that** the first distributor piece (10A) has at least one line (11A, 11B, 11C) for each of the at least two connections (2A, 2B, 2C).

7. The cleaning or care device (1) according to Claim 6, **characterized in that** the second distributor piece (10B) has at least one line (12) for a cleaning or care agent, wherein by moving the first distributor piece (10A) in relation to the second distributor piece (10B) the lines (11 A, 11 B, 11 C) of the first distributor piece (10A) can be connected to the at least one line (12) of the second distributor piece (10B).

8. The cleaning or care device (1) according to any one of Claims 5 - 7, **characterized by** disk-shaped first and second distributor pieces (10A, 10B), wherein each distributor piece (10A, 10B) has an outer lateral surface (13A, 14A) and two cover faces (13B, 13C; 14B, 14C) and wherein the two distributor pieces (10A, 10B) are arranged such that they slide against one another with one cover surface each (13C, 14B).

9. The cleaning or care device (1) according to any one of Claims 2 - 8, **characterized by** a detection unit (15) for positioning the base element (5) in a predetermined starting position.

10. The cleaning or care device (1) according to Claim 9, **characterized in that** the detection unit (15) comprises a marking (16) on a drive element (7) which connects the drive motor (6) to the base element (5) and a sensor (17) which detects the marking (16).

11. The cleaning or care device (1) according to any one of the preceding claims, **characterized by**
a detection unit (18) for determining which of the at least two connections (2A, 2B, 2C) is occupied by a medical, in particular dental, instrument and/or which of the at least two connections (2A, 2B, 2C) is not occupied by a medical, in particular dental, instrument and for delivering the corresponding occupancy signals, wherein the detection unit (18) is connected to a control device (19), which is designed on the basis of the occupancy signals delivered by the detection unit (18), to supply cleaning or care agent only to that one of the at least two connections (2A, 2B, 2C) to which a medical, in particular dental, instrument is connected.

12. The cleaning or care device (1) according to any one of the preceding claims, **characterized in that**
nozzles (20) for dispensing a cleaning or care agent to the outside of the medical, in particular dental, instruments are provided separately arranged from the at least two connections (2A, 2B, 2C).

13. A method for cleaning or care of medical, in particular dental, instruments, with a cleaning or care device (1) according to any one of the preceding claims, **characterized in that**
one of the at least two connections (2A, 2B, 2C) is connected to the supply device (3) and is supplied with a cleaning or care agent by the supply device (3), while another of the at least two connections (2A, 2B, 2C) is not connected to the supply device (3) and does not receive any cleaning or care agent from the supply device (3).

14. The method for cleaning or care of medical, in particular dental, instruments according to Claim 13, **characterized in that**
the at least two connections (2A, 2B, 2C) are connected to the supply device (3) and are supplied with a cleaning or care agent by the supply device (3) one after the other so that at least a portion of the cleaning or care method, in particular the cleaning or care of the interior of the medical, in particular dental, instruments proceeds sequentially.

15. The method for cleaning or care of medical, in particular dental, instruments according to Claim 13 or 14, **characterized in that**
only these of the at least two connections (2A, 2B, 2C), which are occupied by a medical, in particular, dental, instrument, are connected to the supply device (3) and are supplied with a cleaning or care agent by the supply device (3).

## Revendications

1. Appareil de nettoyage ou d'entretien (1) destiné au nettoyage ou à l'entretien d'instruments médicaux, en particulier dentaires, comprenant une chambre de nettoyage ou d'entretien (4), dans laquelle sont prévus au moins deux raccordements (2A, 2B, 2C) pour des instruments à nettoyer, et un dispositif d'alimentation (3) pour alimenter les au moins deux raccordements (2A, 2B, 2C) avec un moyen de nettoyage ou d'entretien, sachant que le dispositif d'alimentation (3) présente un point de transfert pour la liaison au choix avec un des au moins deux raccordements (2A, 2B, 2C) sur lequel le/les moyen(s) de nettoyage ou d'entretien est/sont transféré(s), sachant que sur ce point de transfert, plusieurs conduites (26A, 26B) sont prévues pour les moyens de nettoyage ou d'entretien, sachant que ces conduites (26A, 26B) se terminent dans des ouvertures (34) des au moins deux raccordements (2A, 2B, 2C) auxquelles des conduites d'agent des instruments médicaux, en particulier dentaires, peuvent être couplées, de façon à ce que le moyen de nettoyage ou d'entretien puisse être transporté dans l'intérieur des instruments médicaux, en particulier dentaires, **caractérisé en ce que**,
les au moins deux raccordements (2A, 2B, 2C) et le dispositif d'alimentation (3) sont ainsi mobiles l'un par rapport à l'autre que l'un des au moins deux raccordements (2A, 2B, 2C) est relié au dispositif d'alimentation (3) et peut être alimenté avec un moyen de nettoyage ou d'entretien, pendant qu'un autre des au moins deux raccordements (2A, 2B, 2C) n'est pas relié au dispositif d'alimentation (3) et ne peut pas être alimenté avec un moyen de nettoyage ou d'entretien.

2. Appareil de nettoyage ou d'entretien (1) selon la revendication 1, **caractérisé en ce que**,
les au moins deux raccordements (2A, 2B, 2C) sont disposés sur un élément de base commun (5) qui est mobile par rapport au dispositif d'alimentation (3) de sorte qu'un des au moins deux raccordements (2A, 2B, 2C) soit relié au dispositif d'alimentation (3), pendant qu'un autre des au moins deux raccordements (2A, 2B, 2C) n'est pas relié au dispositif d'alimentation (3).

3. Appareil de nettoyage ou d'entretien (1) selon la revendication 1 ou 2, **caractérisé par** un moteur d'entraînement (6) disposé en-dehors de la chambre de nettoyage ou d'entretien (4), par exemple un moteur électrique, en particulier un moteur pas à pas, pour déplacer les au moins deux raccordements (2A, 2B, 2C) et le dispositif d'alimentation (3) l'un par rapport à l'autre, en particulier pour entraîner l'élément de base (5).

4. Appareil de nettoyage ou d'entretien (1) selon la revendication 3, **caractérisé en ce que**,
le moteur d'entraînement (6) est relié par au moins un élément d'entrainement (7), par exemple un entraînement à courroie (8) et / ou un arbre (9), aux au moins deux raccordements (2A, 2B, 2C) et / ou au dispositif d'alimentation (3) et / ou à l'élément de base (5).

5. Appareil de nettoyage ou d'entretien (1) selon l'une des revendications 2 - 4, **caractérisé par**
un élément distributeur (10) prévu entre l'élément de base (5) et le dispositif d'alimentation (3), pour conduire le moyen de nettoyage ou d'entretien du dispositif d'alimentation (3) vers les au moins deux raccordements (2A, 2B, 2C), sachant que l'élément distributeur (10) présente une première pièce distributrice (10A) mobile avec l'élément de base (5) par rapport au dispositif d'alimentation (3) et une deuxième pièce distributrice (10A) immobile par rapport au dispositif d'alimentation (3).

6. Appareil de nettoyage ou d'entretien (1) selon la revendication 5, **caractérisé en ce que**,
la première pièce distributrice (10A) présente au moins respectivement sa propre conduite (11 A, 11 B, 11 C) pour chacun des au moins deux raccordements (2A, 2B, 2C).

7. Appareil de nettoyage ou d'entretien (1) selon la revendication 6, **caractérisé en ce que**,
la deuxième pièce distributrice (10B) présente au moins une conduite (12) pour un moyen de nettoyage ou d'entretien, sachant que le mouvement de la première pièce distributrice (10A) par rapport à la deuxième pièce distributrice (10B) permet aux conduites (11A, 11B, 11C) de la première pièce distributrice (10A) d'être reliées à l'au moins une conduite (12) de la deuxième pièce distributrice (10B).

8. Appareil de nettoyage ou d'entretien (1) selon l'une des revendications 5 - 7, **caractérisé par**
des première et deuxième pièces distributrices en forme de disque (10A, 10B), sachant que chaque pièce distributrice (10A, 10B) présente une surface d'enveloppe extérieure (13A, 14A) et deux surfaces de couverture (13B, 13C ; 14B, 14C) et sachant que les deux pièces distributrices (10A, 10B) sont disposées en glissant l'une sur l'autre avec une surface de couverture (13C, 14B) respective.

9. Appareil de nettoyage ou d'entretien (1) selon l'une des revendications 2 - 8, **caractérisé par**
une unité de détection (15) pour le positionnement de l'élément de base (5) dans une position de départ prédéterminée.

10. Appareil de nettoyage ou d'entretien (1) selon la revendication 9, **caractérisé en ce que**,
l'unité de détection (15) comprend un marquage (16) sur un élément d'entraînement (7) reliant le moteur d'entraînement (6) à l'élément de base (5), et un capteur (17) détectant le marquage (16).

11. Appareil de nettoyage ou d'entretien (1) selon l'une des revendications précédentes, **caractérisé par**
une unité de détection (18) pour déterminer lequel des au moins deux raccordements (2A, 2B, 2C) est occupé par un instrument médical, en particulier dentaire, et / ou lequel des au moins deux raccordements (2A, 2B, 2C) n'est pas occupé par un instrument médical, en particulier dentaire, et pour émettre des signaux d'occupation correspondants, sachant que l'unité de détection (18) est reliée à un dispositif de commande (19) qui est conçu sur la base des signaux d'occupation fournis par l'unité de détection (18), pour alimenter avec un moyen de nettoyage ou d'entretien celui des au moins deux raccordements (2A, 2B, 2C) auquel un instrument médical, en particulier dentaire, est raccordé.

12. Appareil de nettoyage ou d'entretien (1) selon l'une des revendications précédentes, **caractérisé en ce que**
des buses (20) sont prévues, disposées séparément des au moins deux raccordements (2A, 2B, 2C), pour fournir un moyen de nettoyage ou d'entretien sur le côté extérieur de l'instrument médical, en particulier dentaire.

13. Procédé de nettoyage ou d'entretien d'instruments médicaux, en particulier dentaires, comprenant un appareil de nettoyage ou d'entretien (1) selon l'une des revendications précédentes, **caractérisé en ce que**,
l'un des au moins deux raccordements (2A, 2B, 2C) est relié au dispositif d'alimentation (3) et est alimenté par le dispositif d'alimentation (3) avec un moyen de nettoyage ou d'entretien, pendant qu'un autre des au moins deux raccordements (2A, 2B, 2C) n'est pas relié au dispositif d'alimentation (3) et ne reçoit pas de moyen de nettoyage ou d'entretien du dispositif d'alimentation (3).

14. Procédé de nettoyage ou d'entretien d'instruments médicaux, en particulier dentaires selon la revendication 13, **caractérisé en ce que**,
les au moins deux raccordements (2A, 2B, 2C) sont reliés au dispositif d'alimentation (3) et sont alimentés par le dispositif d'alimentation (3) avec un moyen de nettoyage ou d'entretien l'un après l'autre, de façon à ce qu'au moins une partie du procédé de nettoyage ou d'entretien, en particulier le nettoyage ou l'entretien de l'intérieur des instruments médicaux, en particulier dentaires, s'effectue de manière séquentielle.

15. Procédé de nettoyage ou d'entretien d'instruments médicaux, en particulier dentaires selon la revendication 13 ou 14, **caractérisé en ce que**
seul celui des au moins deux raccordements (2A, 2B, 2C) qui est occupé par un instrument médical, en particulier dentaire, est raccordé au dispositif d'alimentation (3) et alimenté par le dispositif d'alimentation (3) avec un moyen de nettoyage ou d'entretien.
